# EUROPÄISCHE PATENTSCHRIFT

(11) **EP 1 964 466 B1**
(45) Veröffentlichungstag und Bekanntmachung des Hinweises auf die Patenterteilung: **13.04.2011**
(21) Anmeldenummer: 08003074.5
(22) Anmeldetag: 20.02.2008
(51) Int. Cl.: A01K 1/00, A61L 9/012, A61L 9/04, A61L 9/12, A61L 9/14, F24F 3/12

(54) **Verfahren zur Steigerung der Gewichtszunahme bei der Massentierhaltung**
Method for increasing the fattening in intensive animal farming
Méthode pour augmenter l'engraissement lors de l'élevage d'animaux en masse

(30) Priorität: 22.02.2007 DE 102007008720
(43) Veröffentlichungstag der Anmeldung: 03.09.2008
(73) Patentinhaber: Air & D- Sarl, 67190 Rosheim (FR)
(72) Erfinder: Wuest, Robert, 67190 Rosheim (FR)
(74) Vertreter: Ziegler, Stefan Michael

(56) Entgegenhaltungen:
- WO-A-97/19598
- WO-A-98/27261
- WO-A-2004/045657
- DE-A1- 3 434 745
- GB-A- 2 346 900

## Beschreibung

Die Erfindung betrifft ein Verfahren zur Steigerung der Gewichtszunahme von Tieren, insbesondere von Schweinen und von Geflügel in Massentierzuchtanlagen durch Behandeln der Luft in den Anlagen mit aktiven Agenzien.

Nutztiere, insbesondere solche, die zur Ernährung des Menschen bestimmt sind, werden in zunehmendem Maße in Massentierzuchtanlagen gehalten. Bedingt durch die Enge der Anlagen und wohl auch durch die dort herrschende übelriechende Atmosphäre kommt es immer wieder zu hohem Stress bei den Tieren, zu Verletzungen, insbesondere zu Lungenkrankheiten, ja sogar zu Kannibalismus. Außerdem wird das Wachstum und die Gewichtszunahme der Tiere gestört.

Die WO 97/19598 offenbart ein Verfahren nach dem Oberbegriff von Anspruch 1.

Der Erfindung lag die Aufgabe zugrunde, ein alternatives Verfahren anzugeben.

Diese Aufgabe wurde durch das Verfahren nach Anspruch 1 gelöst.

Bei den aktiven Agenzien handelt es sich um natürliche oder synthetische ölige Essenzen. Ihr Siedepunkt liegt im allgemeinen oberhalb von 200 °C, sie können jedoch durch einen Luftstrom zum Verdunsten bzw. Sublimieren gebracht werden. Sie sind meist mit Kohlenwasserstoffen mischbar und in Wasser emulgierbar.

Jasmonal H ist eine blassgelbe Flüssigkeit mit der chemischen Bezeichnung Alpha- Hexylcinnemaldehyd , die bei 305 °C siedet.

Jasmonal H sollte in der Mischung der aktiven Agenzien vorzugsweise zu mehr als 30 Gew.-% enthalten sein.

Thymol hat die chemischen Bezeichnung 2-Isopropyl-5-methyl-phenol. Es handelt sich um farblose Kristalle, die bei 50 °C schmelzen und bei 223 °C sieden. Thymol ist in der Mischung der aktiven Agenzien zu mehr als 30 Gew.-% enthalten.

Bei dem erfindungsgemäßen Verfahren wird die aktive Mischung aus Jasmonal H und dem Thymol vorzugsweise im Gewichtsverhältnis 30 : 70 bis 70 : 30, insbesondere 40 : 60 bis 60 : 40 eingesetzt. Neben Jasmonal H und Thymol können die aktiven Agenzien in untergeordneten Mengen auch noch andere hochsiedende Substanzen, wie z.B. Ketone, Alkohole, Terpene und Ester enthalten. Die aktiven Agenzien kommen zweckmäßigerweise als hydrophobe Mischung mit Verdünnungsmitteln, z.B. mit Kohlenwasserstoffen zur Anwendung, wobei auf 100 Gew.-Teile der aktiven Mischung 5 bis 50, vorzugsweise 10 bis 40 Gew.-Teile des Kohlenwasserstoffs kommen. Bevorzugtes Verdünnungsmittel ist EXXSOL D 40, ein Gemisch von Kohlenwasserstoffen, das im Bereich zwischen 116 und 197 °C siedet.

Die aktive Mischung kann auch als hydrophile wässrige Emulsion zur Anwendung kommen, insbesondere dann, wenn sie direkt in die Tierzuchtanlage hinein micronisiert, d.h. versprüht oder zerstäubt werden soll. Als Emulgierhilfsmittel dienen dann z.B. Alkohole, z.B. DOWANOL, ein Ethoxyethoxyethanol, und/oder Surfectant LRI der Fa. Wacker, eine Mischung aus Polypropylenglykol, Polyethylenglykol und Butylethylglykol. Eine solche Emulsion enthält z.B. 10 bis 30 Gew.-% der aktiven Agenzien und 1 bis 5 Gew.-% eines Emulgierhilfsmittels. Vor dem Einsatz kann diese Emulsion mit 10 bis 100 Gew.-Teilen Wasser verdünnt werden.

Durch die erfindungsgemäße Behandlung der Luft wird - vermutlich durch die Verringerung des Gehalts der Luft an Ammoniak - das Wohlbefinden der Tiere verbessert und der Stress verringert. Dadurch wird ihr Wachstum und die Gewichtszunahme erhöht wird, außerdem wird die Mortalitätsrate durch Krankheiten und Kannibalismus vermindert. Das erfindungsgemäße Verfahren eignet sich daher auch zur Verbesserung der Gesundheit.

Es hat sich gezeigt, dass durch die Behandlung auch der üble Geruch in den Anlagen signifikant verringert wird. Dadurch kann die Frischluftzufuhr vermindert werden, was zu Energieeinsparung besonders in den kalten Monaten führt.

Bei einer bevorzugten Ausführungsform des erfindungsgemäßen Verfahrens wird die aktive Mischung, die zweckmäßigerweise mit einem Kohlenwasserstoff verdünnt ist, in einer Matrix aus einem vernetzten, hydrophile Gruppen enthaltenden Polymeren verteilt, mit dem zusammen es eine gelartige Masse bildet, aus der das aktive Agens langsam freigesetzt wird, verdunstet und in der Gasphase seine Aktivität entfaltet. Bevorzugtes Polymer ist ein Kondensationsprodukt aus einem maleinisierten oder epoxidierten Polymeren, vorzugsweise ein maleinisiertes Polybutadien, und einem Vernetzer, vorzugsweise einem Polyamin. Daneben sind auch Copolymerisate aus einem monofunktionellen (Meth-)Acrylat- Monomeren und einem polyfunktionellen (Meth-)Acrylat- Monomeren geeignet. Bevorzugt wird das aktive Agens bei der Herstellung des Polymeren durch Kondensation oder Polymerisation zugesetzt. Die gelartige Masse kommt vor allem in Form von Platten oder Streifen zur Anwendung, die auf Gitter oder Netze aufgelegt sind, welche in ein offenes Gehäuse eingebracht sind. Dieses kann fünf bis 10 Gelplatten enthalten, wobei die Platten etwa 5 bis 30 cm breit, 8 bis 40 cm lang und 1 bis 6 cm dick sind.

Natürlich kann man die aktive Mischung auch auf andersartige Trägermaterialien, z.B. saugfähige Papiere, hochporöse Tongranulate oder Pulver aufbringen. Das Freisetzen der aktiven Mischung aus dem Trägermaterial, z.B. dem Gel, wird durch einen natürlichen oder vorzugsweise künstlichen Luftstrom, der über das Gel streicht, unterstützt.

Bei einer bevorzugten Ausführungsform der Erfindung werden Gelplatten in ein Gehäuse eingelegt, das an der Decke des Stalls aufgehängt ist. Das Gehäuse ist mit einem Ventilator verbunden, der die verunreinigte Luft aus dem Stall ansaugt. Die Luft streicht dann relativ rasch an den Gelplatten vorbei und desorbiert die aktiven Agenzien. Die mit den aktiven Agenzien beladene Luft wird wieder aus dem Gehäuse ausgeblasen und so im Kreislauf geführt.

Die mit den aktiven Agenzien beladene Gelplatten können einige Wochen bis mehrere Monate lang ihre Wirksamkeit entfalten, bevor sie ausgewechselt werden müssen. Die Platten sind zweckmäßigerweise von einem luftdurchlässigen Stoffvlies umhüllt, um sie vor Staub zu schützen. Es genügt, wenn der Staub z.B. einmal im Monat mechanisch abgeschüttelt wird.

Bei einer anderen Ausführungsform der Erfindung wird das aktive Agens, vorzugsweise als wässrige Emulsion oder in Mischung mit Verdünnungsmitteln, z.B. nach dem Venturi- Prinzip direkt in die Tierzuchtanlage hinein versprüht oder zerstäubt.

### Beispiel 1

35 g maleinisiertes Polybutadien (Umsetzungsprodukt von flüssigem Polybutadien mit Maleinsäureanhydrid - LITHENE der Fa. Revertex), 26 g Jasmonal H, 41 g Thymol, 21 g einer Mischung aus Citral, Eugenol, Geraniol, Alpha- Pinen, Terpen und Terpineol, 12 g EXXSOL D 40, 1 g Wasser und 3 g Polyoxypropylentriamin (MG 440) wurden zusammengerührt.

Die erhaltene gelartige Masse wurde in Platten von 2 cm Dicke, 20 cm Breite und 30 cm Länge geschnitten und auf Metallgitter aufgelegt. Acht Gitter wurden übereinander auf einem Gestell angeordnet, das an der Stalldecke aufgehängt war. Das Gestell war an seiner Unterseite mit einem Ventilator verbunden, der verunreinigte Luft ansaugte und über die Gelplatten streichen ließ. Die Vorrichtung entfaltete über mehr als zwei Monate hinweg ihre Wirkung.

Zu Beginn des Langzeitversuches betrug die tägliche Gewichtszunahme der Schweine im Mittel 586 g und der Ausfall durch Tod betrug im Mittel 1,4 Stück. Nach einem Monat, bedingt durch die erfindungsgemäße Desodorierung, betrug die Gewichtszunahme 704 g und der Ausfall nur 0,92 Stück.

### Beispiel 2

Eine Emulsion wurde hergestellt aus 20 Gew.-Teilen einer Mischung von Jasmonal H und Thymol im Gewichtsverhältnis 51 : 49, 3 Gew.Teilen Surfectant LRI und 77 Gew.teilen Wasser. 5 Gew.teile dieser Emulsion wurden mit 95 Gew.-Teilen Wasser verdünnt und direkt in eine Hühnerbatterie eingesprüht. Die gleiche, mit Wasser verdünnte Emulsion wurde in einen Schweinestall eingesprüht.

## Patentansprüche

1. Verfahren zur Steigerung der Gewichtszunahme von Tieren in Massentierzuchtanlagen durch Behandeln der Luft in den Anlagen mit aktiven Agenzien, **dadurch gekennzeichnet, dass** die aktiven Agenzien eine Mischung aus mindestens 26g Jasmonal H je 88g aktive Agenzien und mehr als 30Gew.-% Thymol enthalten.

2. Verfahren nach Anspruch 1, **dadurch gekennzeichnet, dass** die aktiven Agenzien darüber hinaus in untergeordneten Mengen Ketone, Alkohole, Terpene und Ester enthalten.

3. Verfahren nach Anspruch 1, **dadurch gekennzeichnet, dass** Jasmonal H Thymol und im Gewichtsverhältnis von 30 : 70 bis 70 : 30 eingesetzt werden.

4. Verfahren nach einem der Ansprüche 1 bis 3, **dadurch gekennzeichnet, dass** die aktive Mischung in einem Trägermaterial aufgebracht ist, aus dem es langsam freigesetzt wird und verdunstet.

5. Verfahren nach Anspruch 4, **dadurch gekennzeichnet, dass** die aktive Mischung in einer Matrix aus einem vernetzten, hydrophile Gruppen enthaltenden Polymeren verteilt ist, mit dem zusammen es eine gelartige Masse bildet, aus der das aktive Agens langsam freigesetzt wird und verdunstet.

6. Verfahren nach Anspruch 5, **dadurch gekennzeichnet, dass** die gelartige Masse in Form von Platten oder Streifen auf Gitter oder Netze aufgelegt ist, die in einem offenen Gehäuse eingebracht sind, welches an der Decke der Tierzuchtanlage aufgehängt ist.

7. Verfahren nach Anspruch 6, **dadurch gekennzeichnet, dass** das Gehäuse mit einem Ventilator verbunden ist, der verunreinigte Luft ansaugt, an den Gelplatten vorbei streichen lässt und die mit den aktiven Agenzien beladene Luft wieder ausbläst.

8. Verfahren nach Anspruch 1, **dadurch gekennzeichnet, dass** die aktive Mischung zusammen mit einem Kohlenwasserstoff- Lösungsmittel eingesetzt wird, wobei auf 100 Gew.teile der aktiven Mischung 5 bis 50 Gew.teile des Lösungsmittels eingesetzt werden.

9. Verfahren nach Anspruch 1, **dadurch gekennzeichnet, dass** die aktive Mischung als hydrophile wässrige Emulsion oder als ölige, hydrophobe Mischung mit Verdünnungsmitteln eingesetzt wird.

10. Verfahren nach Anspruch 9, **dadurch gekennzeichnet, dass** die aktive Mischung direkt in die Tierzuchtanlage hinein versprüht oder zerstäubt wird.

## Claims

1. Method for increasing the fattening of animals in intensive animal breeding installations by processing the air in the installations with active agents, **characterised in that** the active agents contain a mixture of at least 26 g jasmonal H per 88 g active agents and more than 30 wt.% thymol.

2. Method according to claim 1, **characterised in that** the active agents additionally contain in lower amounts ketones, alcohols, terpenes and esters.

3. Method according to claim 1, **characterised in that** jasmonal H and thymol are used in a weight ratio of 30 : 70 to 70: 30.

4. Method according to any one of claims 1 to 3, **characterised in that** the active mixture is applied in a carrier material from which it is slowly released and vaporised.

5. Method according to claim 4, **characterised in that** the active mixture is distributed in a matrix from a polymer containing cross-linked, hydrophilic groups, together with which polymer it forms a gel-like mass, from which the active agent is slowly released and evaporated.

6. Method according to claim 5, **characterised in that** the gel-like mass is applied in the form of plates or strips on grids or networks, which are introduced in an open housing which is suspended from the ceiling of the animal breeding installation.

7. Method according to claim 6, **characterised in that** the housing is connected to a ventilator which suctions contaminated air, sweeps past the gel plates and blows out air charged with the active agents.

8. Method according to claim 1, **characterised in that** the active mixture is used together with a hydrocarbon solvent, in which for 100 wt. parts of active mixture 5 to 50 wt. parts of the solvent are used.

9. Method according to claim 1, **characterised in that** the active mixture is used as a hydrophilic, aqueous emulsion or as an oily, hydrophobic mixture with diluents.

10. Method according to claim 9, **characterised in that** the active mixture is sprayed or atomised directly into the animal breeding installation.

## Revendications

1. Procédé permettant d'augmenter l'engraissement d'animaux dans des installations d'élevage en masse par traitement de l'air présent dans les installations avec des agents actifs, **caractérisé en ce que** les agents actifs contiennent un mélange d'au moins 26 g de jasmonal H pour 88 g d'agents actifs et plus de 30 % en poids de thymol.

2. Procédé selon la revendication 1, **caractérisé en ce que** les agents actifs contiennent en outre dans des quantités plus faibles des cétones, des alcools, des terpènes et des esters.

3. Procédé selon la revendication 1, **caractérisé en ce que** le jasmonal H et le thymol sont utilisés selon un rapport de poids de 30:70 à 70:30.

4. Procédé selon l'une des revendications 1 à 3, **caractérisé en ce que** le mélange actif est appliqué dans un matériau de support, depuis lequel il est libéré lentement et volatilisé.

5. Procédé selon la revendication 4, **caractérisé en ce que** le mélange actif est réparti dans une matrice composée d'un polymère réticulé contenant des groupes hydrophiles, avec lequel il est formé conjointement une masse de type gel à partir de laquelle l'agent actif est libéré lentement et volatilisé.

6. Procédé selon la revendication 5, **caractérisé en ce que** la masse de type gel est déposée sous la forme de plaques ou de bandes sur des grilles ou des maillages qui sont introduits dans un boîtier ouvert, ce dernier étant suspendu au plafond de l'installation d'élevage d'animaux.

7. Procédé selon la revendication 6, **caractérisé en ce que** le boîtier est relié à un ventilateur qui aspire l'air chargé d'impuretés, le balaie au niveau des plaques de gel et souffle à nouveau l'air chargé en agent actif.

8. Procédé selon la revendication 1, **caractérisé en ce que** le mélange actif est utilisé avec un solvant d'hydrocarbure, où pour 100 parties en poids du mélange actif, on utilise 5 à 50 parties en poids du solvant.

9. Procédé selon la revendication 1, **caractérisé en ce que** le mélange actif est utilisé en tant qu'émulsion aqueuse hydrophile ou en tant que mélange huileux hydrophobe avec des diluants.

10. Procédé selon la revendication 9, **caractérisé en ce que** le mélange actif est directement pulvérisé ou atomisé dans l'installation d'élevage d'animaux.
